# EUROPEAN PATENT APPLICATION

(11) **EP 4 424 816 A1**
(43) Date of publication of application: **04.09.2024**
(21) Application number: 22887175.2
(22) Date of filing: 28.10.2022
(51) Int. Cl.: C12N 5/07, C12N 1/02, C12Q 1/24

(54) **CELL DETACHMENT SOLUTION, CELL DETACHMENT METHOD, AND CELL PRESERVATION METHOD**

(30) Priority: 29.10.2021 JP 2021177910
(71) Applicant: CANON KABUSHIKI KAISHA, Tokyo 146-8501 (JP)
(72) Inventor: MOCHIZUKI Shinsuke, Tokyo 146-8501 (JP); KATO Mai, Tokyo 146-8501 (JP); TSUBAKI Keiichiro, Tokyo 146-8501 (JP); TOMATSU Fumiko, Tokyo 146-8501 (JP); YAMAUCHI Fumio, Tokyo 146-8501 (JP); KANAZAKI Kengo, Tokyo 146-8501 (JP)
(74) Representative: TBK
(86) International application number: PCT/JP2022/040387
(87) International publication number: WO 2023/074850

(57) **Abstract**

A cell dissociation solution is used in dissociation of, from a substrate, a cell disposed on the substrate, by application of ultrasonic vibration to the cell and the cell dissociation solution contains a polymer including a polyalkylene glycol structure.

## Description

### Technical Field

The present invention relates to a cell dissociation solution, a cell dissociation method, and a cell preservation method.

### Background Art

In the fields of cell therapy products and regenerative therapy, large amounts of cells are required and, in particular, efficient and stable supply of adherent cells, which largely constitute living tissues, has been in demand.

In culture of adherent cells, for example, steps of performing culture of cells on a culture substrate such as a polystyrene dish, dissociation of the cells from the substrate, and collection and washing of the cells are performed, to obtain the target cells. When the cells are further grown, a portion of the obtained cells are transferred to a new substrate and cultured, which is called, the subculture operation. In such a series of steps, the dissociation step of dissociating cells from the substrate is an obstacle to efficient and stable supply of cells.

On the other hand, cell dissociation methods using ultrasonic waves have been proposed. Patent Literature 1 discloses a method in which a culture vessel including adherent cells cultured using a medium is turned upside down and ultrasonic vibration is applied to the culture vessel from the outer surface of the culture vessel, to thereby dissociate the cells from the cell adhesion surface. Patent Literature 2 discloses a cell dissociation apparatus that irradiates a vessel holding cells and a medium or a fluid with ultrasonic waves to dissociate at least a portion of the cells from the vessel.

The inventors of the present invention have found drawbacks in the above-described related art. Specifically, for example, in the case of treating cells that are less likely to be dissociated, such as cells having high adhesiveness to vessels, dissociation performed under ultrasonic vibration conditions providing higher dissociation efficiency may result in a decrease in the cell viability.

### Citation List

### Patent Literature

PTL 1: Japanese Patent Laid-Open No. 2006-314204
PTL 2: International Publication No. 2016-047368

### Summary of Invention

### Technical Problem

Accordingly, an object of the present invention is to provide a cell dissociation solution that is used for cell dissociation using ultrasonic waves and provides both of a high cell viability and a high dissociation ratio, and a cell dissociation method and a cell preservation method that use the cell dissociation solution.

### Solution to Problem

A cell dissociation solution according to the present invention is a cell dissociation solution used in dissociation of, from a substrate, a cell disposed on the substrate, by application of ultrasonic vibration to the cell, the cell dissociation solution containing a polymer including a polyalkylene glycol structure.

### Advantageous Effects of Invention

In the present invention, a cell dissociation solution according to the present invention can provide a cell preservation method in which, in cell dissociation using ultrasonic waves, both of a high cell viability and a high dissociation ratio are provided and furthermore subsequently the cell is simply and effectively preserved in the cell dissociation solution.

### Brief Description of Drawings

[Fig. 1] Fig. 1 is a flow chart illustrating an example of the flow of a cell preservation method according to an embodiment.
[Fig. 2] Fig. 2 is a schematic diagram of a cell preservation apparatus according to an embodiment. Description of Embodiments

Hereinafter, a cell dissociation solution according to an embodiment of the present invention will be described.

The dissociation solution according to the embodiment is a cell dissociation solution used in dissociation of, from a substrate, a cell disposed on the substrate, by application of ultrasonic vibration to the cell. The cell dissociation solution contains a polymer including a polyalkylene glycol structure. The cell dissociation solution contains the polymer including a polyalkylene glycol structure, to thereby increase the cell dissociation ratio and the cell viability in dissociation of the cell under application of ultrasonic vibration. Hereinafter, this will be described in detail. The cell serving as the dissociation target may be a single cell, an aggregate of a plurality of cells, or cells cultured in a sheet form (cell sheet) (hereafter, the same definition).

### (Operation and effect)

The cell dissociation solution according to the embodiment contains a polymer including a polyalkylene glycol structure, to thereby provide an increased cell viability. The inferential reason for this is specifically as follows. In an attempt to dissociate a cell from a substrate by application of ultrasonic vibration, the vibration is applied to the cell to damage the cell in some cases. However, when the polymer including a polyalkylene glycol structure in the cell dissociation solution adsorbs to the cell membrane, a portion of the vibration energy propagates to the polymer, so that large vibration can be prevented from propagating to the cell. The polymer including a polyalkylene glycol structure includes the polyalkylene glycol structure and hence is hydrophilic (can also be referred to as a hydrophilic polymer), so that oxygen in the polyalkylene glycol structure tends to adsorb to the cell membrane. Thus, this inferentially results in improvement in the effect of protecting the cell.

### (Polymer including polyalkylene glycol structure)

In this embodiment, specific examples of the polymer including a polyalkylene glycol structure include polyethylene glycol, polypropylene glycol, polybutylene glycol, copolymers including the foregoing as partial structures, and derivatives such as polyethylene glycol dimethyl ether, polyethylene glycol lauryl ether, and polypropylene glycol butyl ether. When the polymer is a copolymer, the content of the polyalkylene glycol structure is preferably 90 mass% or more.

Of the specific examples, preferred are those having an OH group as an end group and having a polyalkylene glycol structure having a carbon chain having 5 or less carbon atoms from the viewpoint of having high hydrophilicity; of these, more preferably used is polyethylene glycol. Note that, in this Description, polyethylene glycol may be referred to as PEG (polyethylene glycol).

Note that the polymer including a polyalkylene glycol structure may be linear or have a branched structure.

The polymer including a polyalkylene glycol structure used in the embodiment has a peak molecular weight Mp of, as measured by gel permeation chromatography, preferably 800 or more and 50000 or less, more preferably 1200 or more and 20000 or less. This is because, when the polymer has an Mp of 800 or more, the polymer does not permeate the cell; when the polymer has an Mp of 50000 or less, polymer molecules are less likely to repel each other and the polymer can be stably present around the cell membrane. Note that the molecular weight of the polymer can be adjusted by publicly known methods and specifically can be appropriately adjusted by, for example, selecting the ratio of monomers charged, the polymerization time, or the catalyst.

The method of measuring the molecular weight of the polymer in the embodiment will be described later.

### (Cell dissociation solution)

In the embodiment, the polymer content relative to the total mass of the cell dissociation solution is preferably 0.03 mass% or more and 20.0 mass% or less, more preferably 0.05 mass% or more and 5.0 mass% or less. This is because when the polymer content is 0.03 mass% or more, the cell protection effect is sufficiently provided; when the polymer content is 20.0 mass% or less, cells are less likely to aggregate.

The cell dissociation solution according to the embodiment is preferably substantially free from protease. The "substantially free from protease" means that the protease content relative to the total mass of the cell dissociation solution is 0.0005 mass% or less. More preferably, protease is not contained. The reason for this is as follows.

In general, cells are dissociated with proteases such as trypsin, which are widely used. In this method, the action of such a protease degrades the protein contributing to adhesion between such a cell and the substrate and subsequently an operation such as tapping or pipetting is performed to completely dissociate the cell. However, in this method, the protease simultaneously degrades large amounts of cell surface proteins, which may result in degradation of the cell quality. In addition, the operation such as tapping or pipetting provides its effect that considerably varies depending on the person's skill and hence has drawbacks in terms of stable supply. In summary, protease, which degrades a portion of the cell, provides an increase in the dissociation ratio, but may degrade the cell quality (may cause a decrease in the cell viability).

The cell dissociation solution according to the embodiment is preferably substantially free from divalent cations. The "substantially free from divalent cations" means that the cell dissociation solution has a divalent cation concentration of 0.05 µM or less. More preferably, the cell dissociation solution according to the embodiment does not contain divalent cations. This is intended to weaken the action of the divalent cation-dependent adhesion proteins such as integrin, to facilitate a further increase in the dissociation ratio. Furthermore, in order to actively remove the divalent cations from the adhesion protein, a chelating agent is preferably contained. The chelating agent content is preferably 0.01 mM or more and 5.0 mM or less. When this range is satisfied, the chelating effect is more likely to be provided while a decrease in the activity caused by the presence of an excessive amount of chelating agent can be reduced.

The cell dissociation solution according to the embodiment preferably has a pH in the neutral region. This is because the neutral region is suitable for culture of cells and a high cell viability can be stably kept. The pH can be appropriately adjusted using hydrochloric acid or sodium hydroxide, for example. In order to stably keep the pH, various buffer solutions are preferably used.

The cell dissociation solution according to the embodiment preferably has a viscosity of 1.80 mPa·s or less. This is because the flow of the dissociation solution generated by ultrasonic vibration is not hampered and a high dissociation ratio can be kept. The viscosity of the cell dissociation solution can be appropriately adjusted by adjusting, for example, the molecular weight of the polymer or the amount of the polymer added.

### <Protease>

In the embodiment, the protease is a substance that, for example, degrades a portion of the cell to facilitate dissociation of the cell from the substrate. Examples include trypsin, accutase, collagenase, natural protease, chymotrypsin, elastase, papain, pronase, and recombinants of the foregoing.

### <Divalent cations>

Examples of the divalent cations in the embodiment include Ca²⁺, Mg²⁺, Zn²⁺, Co²⁺, Ni²⁺, and Mn²⁺. The adhesion proteins such as integrin have considerably high tendency on Ca²⁺ and Mg²⁺ and hence the cell dissociation solution according to the embodiment is preferably substantially free from Ca²⁺ and Mg²⁺. The "substantially free from" means that the cell dissociation solution has Ca²⁺ and Mg²⁺ concentrations of 0.05 µM or less. More preferably, the cell dissociation solution according to the embodiment does not contain Ca²⁺ or Mg²⁺.

### <Buffer solution>

The buffer solution in the embodiment can be used without limitations as long as it can keep the neutral region. Examples include Tris buffer solutions such as a Tris-HCl buffer solution, a phosphate buffer solution, a HEPES buffer solution, a citrate-phosphate buffer solution, a glycylglycine-sodium hydroxide buffer solution, a Britton-Robinson buffer solution, and a GTA buffer solution. Of these, preferred is the phosphate buffer solution, which is close to the in vivo environment; this phosphate buffer solution is adjusted so as to be isotonic with the intracellular fluid to provide phosphate buffered saline (PBS), which is more preferably used. In the case of using PBS, PBS(-), which is free from divalent cations such as Ca²⁺ and Mg²⁺, is preferably used.

### <Chelating agent>

The chelating agent used in the embodiment is not particularly limited and examples include ethylenediaminetetraacetic acid, ethylenediamine, ethylenediaminetetramethylene phosphonic acid, glycol ether diamine tetraacetic acid, nitrilotriacetic acid, diethylenetriaminepentaacetic acid, iminodiacetic acid, dihydroxyethyl glycine, dicarboxymethylglutamic acid, ethylenediaminedisuccinic acid, etidronic acid, citric acid, gluconic acid, and phosphonobutanetriacetic acid. Of these, preferred are chelating agents that chelate with divalent cations; particularly preferred are chelating agents that chelate with Ca²⁺ and Mg²⁺; most preferred is ethylenediaminetetraacetic acid. In the case of using, as the chelating agent, ethylenediaminetetraacetic acid, the cell dissociation solution preferably has a pH of 7.0 or more and 8.0 or less. This is because, when the pH is a relatively high pH in the neutral region in which a high cell viability can be kept, ethylenediaminetetraacetic acid can have higher chelating activity to provide a further increase in the dissociation ratio. Note that such chelating agents may be used alone or in combination of two or more thereof.

### (Substrate)

The substrate in the embodiment is formed of a material that is chemically stable and on which given cells can be cultured; examples include polyethylene, polypropylene, polycarbonate, polystyrene, polyvinyl chloride, nylon, polyurethane, polyurea, polylactic acid, polyglycolic acid, polyvinyl alcohol, polyvinyl acetate, poly(meth)acrylic acid, poly(meth)acrylic acid derivatives, polyacrylonitrile, poly(meth)acrylamide, poly(meth)acrylamide derivatives, polysulfone, cellulose, cellulose derivatives, polysilicone, polymethylpentene, glass, and metals. Of these, preferred is polystyrene. Note that the substrate may be referred to as a dish.

### (Cell)

The cell in the embodiment is not particularly limited as long as the cell can be cultured in vitro as an adherent cell on a culture material. Examples include various culture cell lines such as Chinese hamster ovary derived CHO cells, mouse connective tissue L929 cells, mouse skeletal muscle myoblast cells (C2C12 cells), human fetal lung-derived normal diploid fibroblast cells (TIG-3 cells), human fetal kidney-derived cells (HEK293 cells), human alveolar basement epithelium adenocarcinoma-derived A549 cells, and human cervical cancer-derived HeLa cells; examples further include cells constituting tissues and organs in the living body such as epithelial cells and endothelial cells, cells exhibiting contractility such as skeletal muscle cells, smooth muscle cells, and cardiac muscle cells, cells constituting the nervous system such as neuron cells, glial cells, and fibroblast cells, cells contributing to metabolism of living bodies such as hepatic parenchymal cells, hepatic nonparenchymal cells, and fat cells, cells having differentiation potency that are various stem cells such as induced pluripotent stem (iPS) cells, embryonic stem (ES) cells, embryonic germ (EG) cells, embryonic carcinoma (EC) cells, mesenchymal stem cells, liver stem cells, pancreatic stem cells, skin stem cells, muscle stem cells, and germ stem cells, precursor cells of tissues, and cells differentiation-induced from the foregoing. Of these, to the cells having high adhesiveness to substrates and cells having high sensitivity to trypsin, the cell dissociation solution according to the embodiment is also suitably applied.

### (Medium)

The type of the medium is not particularly limited and examples include Dulbecco's Modified Eagles's Medium (Dulbecco's Modified Eagles's Medium; DMEM), Ham's Nutrient Mixture F12 (Ham's Nutrient Mixture F12), DMEM/F12 medium, McCoy's 5A medium (McCoy's 5A medium), Eagles's MEM medium (Eagles's Minimum Essential Medium; EMEM), αMEM medium (alpha Modified Eagles's Minimum Essential Medium; αMEM), MEM medium (Minimum Essential Medium), RPMI1640 medium, Iscove's Modified Dulbecco's Medium (Iscove's Modified Dulbecco's Medium; IMDM), MCDB131 medium, Williams' medium E, IPL41 medium, Fischer's medium, StemSpan H3000 (manufactured by STEMCELL Technologies), StemSpanSFEM (manufactured by STEMCELL Technologies), StemlineII (manufactured by Sigma-Aldrich Corporation), Endothelial Cell Growth Medium 2 Kit (manufactured by PromoCell GmbH), Mesenchymal Stem Cell Growth Medium 2 (manufactured by PromoCell GmbH), MSCGM Bullet Kit (manufactured by Lonza), mTeSR1 or 2 medium (manufactured by STEMCELL Technologies), Repro FF or Repro FF2 (manufactured by ReproCELL Inc.), NutriStem medium (manufactured by Biological Industries), and MF-Medium mesenchymal stem cell growth medium (manufactured by TOYOBO CO., LTD.).

Of these, a medium suitable for culture of the given cell is preferably used. For example, for culture of CHO cells, Ham's Nutrient Mixture F12 is suitably used; for C2C12 cells, DMEM/F12 medium is suitably used.

### <Serum>

To the medium, serum or an antibiotic may be added. Examples of the serum include Fetal Bovine Serum (Fetal Bovine Serum: FBS), bovine calf serum, adult bovine serum, horse serum, sheep serum, goat serum, pig serum, chicken serum, rabbit serum, and human serum; because of ease of availability, in general, FBS is often used. Alternatively, a serum-free medium, which does not contain untreated or unpurified serum, but contains purified blood-derived components or animal tissue-derived components (such as a growth factor), may be used.

### <Antibiotic>

Examples of the antibiotic added to the medium include penicillin, streptomycin, ampicillin, carbenicillin, tetracycline, bleomycin, actinomycin, kanamycin, actinomycin D, and amphotericin B.

### (Cell culture conditions)

Cell culture conditions can be appropriately selected in accordance with the cells to be cultured. In general, an appropriate medium is added to a dish; to the dish, cells are seeded at about 1.0 × 10¹ to about 5.0 × 10⁴ cells/cm² and cultured in an environment at 37°C and at a CO₂ concentration of 5%. In this case, the cells are preferably cultured until the confluency in the substrate reaches about 70% to about 80%, in other words, the cells become sub-confluent.

### (Ultrasonic vibration)

In the embodiment, the ultrasonic vibration is, for example, as described in International Publication No. 2016-047368, vibration having a frequency of about 10 kHz to about 1 MHz. The vibration generation means can be used without particular limitations as long as the means can apply ultrasonic vibration to the cell. An example is, as described in Japanese Patent Laid-Open No. 2008-92857, an ultrasonic radiator formed of lead titanate zirconate (PZT).

As described in Japanese Patent Laid-Open No. 2006-314204, a vibrator can be brought into direct contact with the outer surface of a culture vessel in which a medium is charged and sealed, to apply vibration to the culture vessel. Alternatively, as described in International Publication No. 2016-047368, instead of bringing the ultrasonic radiation means into direct contact with the vessel, an ultrasonic transmission substance can be disposed between the ultrasonic radiation means and the treatment target region and ultrasonic waves can be caused to enter the cell serving as the dissociation target.

### (Cell dissociation method)

A cell dissociation method according to an embodiment includes at least the following steps.

### <First step>

A step of preparing a substrate on which a cell and a cell dissociation solution in contact with the cell and the substrate are disposed.

### <Second step>

A step of applying ultrasonic vibration to the cell to dissociate the cell from the substrate.

### <Other step>

The cell dissociation method according to the embodiment may include, in addition to the above-described steps, a step of applying an external stimulus such as convection to the culture solution. The convection in the culture solution can be caused by, for example, pipetting or using a pump or an impeller.

### <Environmental temperature>

The second step is performed at an environmental temperature of preferably 30.0°C or more and 37.5°C or less. This is because the temperature during dissociation is close to the temperature during culture, so that the cell is less likely to be affected by temperature change and a high viability can be kept. In addition, in the dissociation solution having a divalent cation concentration of 0.05 µM or less, when the temperature range is satisfied, divalent cations bonded to the adhesion protein tend to leave and the dissociation ratio also increases.

### (Cell preservation method)

The cell dissociation method according to the embodiment may include, in addition to the above-described steps, a step of preserving the cell.

A cell preservation method according to an embodiment includes at least the following steps.

A step 1 is a step of bringing a cell into contact with the cell dissociation solution according to the embodiment substantially free from the degradation enzyme. The cell and the cell dissociation solution substantially free from the degradation enzyme are brought into contact with each other to thereby reduce intercellular junction and the adhesiveness between the cell and the substrate.

A step 2 is a step of applying ultrasonic vibration to the cell, to dissociate the cell from the substrate.

A step 3 is a step of preserving, in the cell dissociation solution, the cell having been dissociated in the step 2.

In the embodiment, cell preservation means a procedure of leaving, for at least 1 hour or more, a cell living in the cell dissociation solution while keeping the cell alive for the purpose of subsequently using the cell again.

In the cell preservation method according to the embodiment, the cell dissociation solution is used for the cell and ultrasonic vibration is applied to the cell to dissociate the cell, and subsequently the cell is preserved in the cell dissociation solution. In other words, the cell having been dissociated can be subsequently preserved as they are; alternatively, various additives may be appropriately added to the cell dissociation solution after the dissociation to improve the preservation effect. Such an additive for improving the preservation effect may be at least one selected from the group consisting of saccharides, serums, and antibiotics.

Cell preservation according to the embodiment can be performed for the cell suspended in the cell dissociation solution. The cell concentration during preservation can be appropriately selected in accordance with conditions such as the type of the cell, the size of the cell, or the preservation period of the cell; the cell concentration may be, for example, relative to 1 mL of a cell suspension containing the cells and the dissociation solution, in a range of 10000 cells or more and 100000000 cells or less, or 100000 cells or more and 10000000 cells or less.

In the cell preservation method according to the embodiment, the cell preservation period depends on conditions such as the type of the cell, the preservation temperature, the composition of the cell dissociation solution, and the cell concentration, but is preferably in the range of about 1 hour or more and about 10 days or less, preferably 6 hours or more and 7 days or less.

In the cell preservation method according to the embodiment, the cell preservation temperature is, from the viewpoint of preserving an alive cell and culturing again the cell as it is, preferably non-freezing temperature, specifically, in the range of more than 0° and 30°C or less, preferably in the range of 1°C or more and 10°C or less. When the temperature is 0°C or less, the cell is in a cryopreservation state and the cell may be damaged during thawing. In order to prevent this, after the cell is dissociated, a cryoprotective agent may be added to the cell dissociation solution. From the viewpoint of suppressing the metabolic activity of the cell during preservation, the preservation temperature is particularly preferably 2°C or more and 8°C or less. Note that the cell preservation temperature in the embodiment can be the temperature set for the refrigerator in which the cell is preserved.

Specifically, an example of the preservation is a preservation method performed during transport of adherent cells from a culture facility to a medical institution. In this case, the preservation period is in the range of 2 days or more and 3 days or less and the temperature is in the range of 2°C or more and 8°C or less.

In the cell preservation method according to the embodiment, the vessel used for cell preservation can be a publicly known vessel selected in accordance with, for example, the type of the cell, the preservation temperature, and the application of the post-preservation cell. From the viewpoint of, for example, airtightness, cytotoxicity, and sterilization, a tube of polypropylene or polyethylene is preferably used.

An apparatus for the cell preservation is not particularly limited as long as, in the apparatus, the above-described vessel for cell preservation can be left at rest stably and the above-described preservation temperature can be satisfied; the apparatus may be, for example, an incubator or a refrigerator.

### (Example of application after cell preservation)

The embodiment includes a method of using the cell having been preserved in the cell dissociation solution, to culture the cell using an ordinary cell growth medium.

For the cell having been preserved in the above-described manner, the cell dissociation solution containing the cell may be centrifuged and subsequently the supernatant may be removed; the cell may be subsequently dispersed again in an ordinary cell growth medium, seeded on a culture substrate, and cultured. Note that the cell dissociation solution containing the cell may be directly seeded on an ordinary cell growth medium to perform the culture. The cell concentration for resuming the culture varies depending on the type of the cell, but may be, for example, the cell concentration employed during subculture of the cell.

### (Configuration of cell preservation apparatus)

A cell preservation apparatus according to an embodiment is a cell preservation apparatus that is configured to dissociate and collect an adherent cell cultured on a substrate and to preserve the cell, and includes a medium exchange section, an ultrasonic radiation section, and a cell preservation section. The exchange section is configured to add, to the substrate to which the cell adheres, the cell dissociation solution to bring the cell and the cell dissociation solution into contact with each other. The ultrasonic radiation section is configured to apply ultrasonic vibration to the cell. The cell preservation section is configured to preserve, at a constant temperature, the cell having been dissociated from the substrate.

### (Flow of cell preservation method according to embodiment)

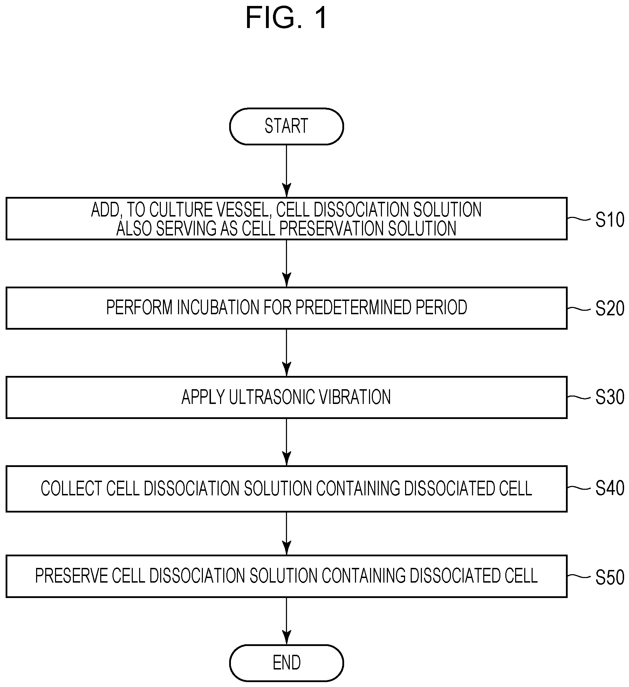

Fig. 1 illustrates an example of the flow of the cell preservation method according to the embodiment. For example, the step 1 includes a step S10 of adding, to a culture vessel, the cell dissociation solution (also serving as a cell preservation solution), and a step S20 of incubating and maintaining, for a predetermined period, the cell and the cell dissociation solution (also serving as a cell preservation solution) that are in contact with each other. The step 2 includes, for example, a step S30 of applying ultrasonic vibration in order to dissociate the cell from the substrate, and may further include a step S40 of collecting the cell dissociation solution (also serving as a cell preservation solution) containing the cell having been dissociated from the substrate. The step 3 includes, for example, S50 of preserving the cell dissociation solution (also serving as a cell preservation solution) containing the cell having been dissociated. The flow of the cell preservation method according to the embodiment may further include a step of observing the state of the cell.

### (Example of application of post-preservation cell)

For the cell having been preserved in the above-described manner, the cell dissociation solution containing the cell (also serving as a cell preservation solution) may be centrifuged and subsequently the supernatant may be removed; the cell may be subsequently dispersed again in an ordinary cell growth medium, seeded on a culture substrate, and cultured. Note that the cell dissociation solution containing the cell (also serving as a cell preservation solution) may be directly seeded on an ordinary cell growth medium to perform the culture. The cell concentration for resuming the culture varies depending on the type of the cell, but may be, for example, the cell concentration employed during subculture of the cell. The embodiment includes a method of using the cell having been preserved in the cell dissociation solution (also serving as a cell preservation solution), to culture the cell using an ordinary cell growth medium.

### (Configuration of cell preservation apparatus)

A cell preservation apparatus according to an embodiment is a cell preservation apparatus that is configured to dissociate and collect an adherent cell cultured on a substrate and to preserve the cell, and includes a medium exchange section, an ultrasonic radiation section, and a cell preservation section. The exchange section is configured to add, to the substrate to which the cell adheres, the cell dissociation solution (also serving as a cell preservation solution) to bring the cell and the cell dissociation solution (also serving as a cell preservation solution) into contact with each other. The ultrasonic radiation section is configured to apply ultrasonic vibration to the substrate and the cell. The cell preservation section is configured to preserve, at a constant temperature, the cell having been dissociated from the substrate.

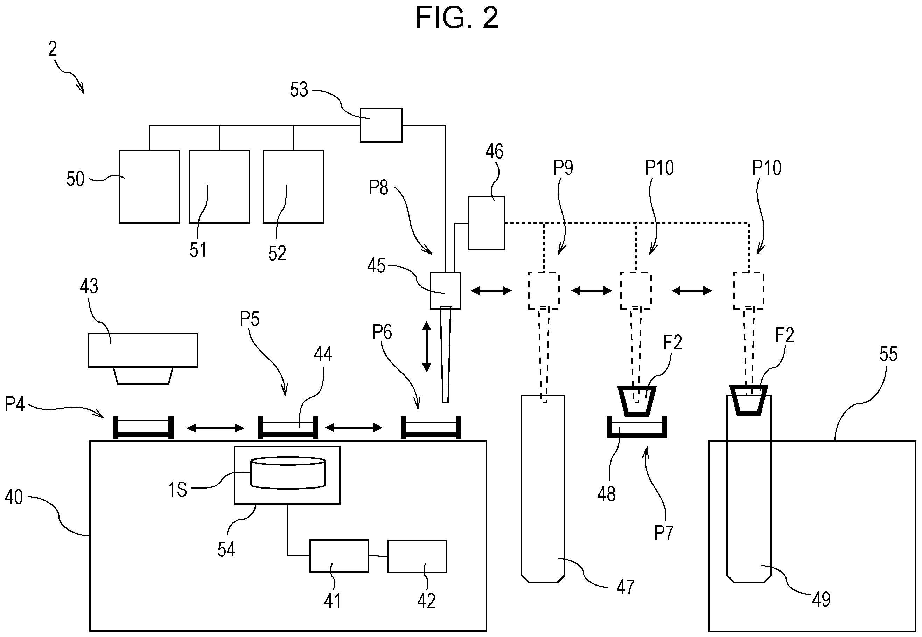

The configuration of a cell preservation apparatus 2 according to the embodiment will be described with reference to the schematic diagram in Fig. 2.

A culture substrate transfer controller 40 is a unit configured to hold and transfer a culture substrate 44.

The culture substrate transfer controller 40 may include a temperature regulator configured to control the temperature and humidity of the environment in which the cell is dissociated from the substrate; examples of the temperature regulator include a heating unit configured to keep the temperature at 37°C and a humidifying unit. In order to transfer the culture substrate 44, for example, an XYZ electric stage is used. The culture substrate transfer controller 40 horizontally transfers the culture substrate 44 among a position P4, a position P5, and a position P6.

At the position P4, an image of the cell cultured within the culture substrate 44 can be captured. At the position P5, into the culture substrate 44, ultrasonic waves are radiated from an ultrasonic radiation section 1S. At the position P6, a pipetting section 45 is used to recover the solution in the culture substrate 44 or to add various solutions to the culture substrate 44.

This will be described further with reference to an example of a cell dissociation process using ultrasonic waves.

A cell is cultured in the culture substrate 44 and, at the timing for dissociation of the cell, the culture substrate 44 is placed at the position P6. The culture substrate transfer controller 40 is appropriately used to place the culture substrate 44 at the position P4, so that a cell observation section 43 can be used to observe the state of the cell. The cell adheres to the culture substrate 44 placed at the position P6.

First, the medium needs to be removed and exchanged for the cell dissociation solution (also serving as a cell preservation solution). Thus, at the position P6, the pipetting section 45 is used to recover the medium in the culture substrate 44. The recovered medium is discarded, at a position P9, to a waste fluid recovery section 47. Various media are stocked in a washing medium 50, a culture medium 51, and a cell dissociation solution (also serving as a cell preservation solution) 52, and are transported by a liquid transport controller 53 to the pipetting section 45. The pipetting section 45 is configured to move vertically to access the culture substrate 44. The cell preservation apparatus 2 may optionally include an open-close system for the lid of the culture substrate 44.

After the medium is removed, the cell adhering to the culture substrate 45 may be optionally washed. The liquid transport controller 53 is used to transport the washing medium 50 to the pipetting section 45 and, at the position P6, the pipetting section 45 is used to add the washing medium 50 into the culture substrate 44. The washing medium 50 is removed as in the above-described method of removing the medium and subsequently the liquid transport controller 53 is used to transport the cell dissociation solution and cell preservation solution 52 to the pipetting section 45. Subsequently, at the position P6, the pipetting section 45 is used to add, into the culture substrate 44, the cell dissociation solution (also serving as the cell preservation solution) 52.

Subsequently, the culture substrate transfer controller 40 transfers the culture substrate 44 to the position P5, where the culture substrate 44 is set to the ultrasonic radiation section 1S. For the ultrasonic radiation section 1S, a publicly known configuration can be employed. The configuration including the ultrasonic radiation section 1S is, for example, a structure of a combination of an ultrasonic dissociation unit 54 including the ultrasonic radiation section 1S and a function generator 42 and an amplifier 41 that are configured to input, for example, a given frequency and a given voltage to the ultrasonic dissociation unit 54.

In the cell preservation according to the embodiment, the culture substrate 44 is irradiated with ultrasonic waves from the ultrasonic radiation section 1S, to dissociate the cell adhering to the culture substrate 44. The culture substrate transfer controller 40 transfers the culture substrate 44 to the position P6. At the position P6, the pipetting section 45 is used to collect, from the culture substrate 44, the cell dissociation solution (also serving as the cell preservation solution) containing the cell having been dissociated from the culture substrate 44. Subsequently, at a position P10, the cell dissociation solution (also serving as the cell preservation solution) containing the cell is passed through a mesh filter F2 (not necessarily used when such cells are sufficiently dispersed) and collected to a dissociated cell preservation vessel (tube) 49 disposed in a cell preservation section 55. The cell preservation section 55 includes a mechanism configured to adjust the temperature, and can keep, at a constant temperature (cool), the cell dissociation solution (also serving as the cell preservation solution) containing the cell. In this state, the cell can be preserved. The cell preservation apparatus 2 may optionally include an open-close system for the lid of the dissociated cell preservation vessel (tube) 49.

In addition to the cell preservation, a portion of such dissociated cells may be seeded in a dissociated cell collection section 48, for example, a new cell culture substrate disposed at a position P7. A portion of the cells collected here can be used for an assay such as measurement of the cells.

The cell after a predetermined preservation period can be used again (for example, cultured again). For example, at the position P10, the pipetting section 45 is used to collect the cell preserved in the dissociated cell preservation vessel (tube) 49. Subsequently, at the position P10, the cell dissociation solution (also serving as the cell preservation solution) containing the cell is passed through the mesh filter F2 (not necessarily used when such cells are sufficiently dispersed). Subsequently, the cell can be seeded to the dissociated cell collection section 48, for example, a new cell culture substrate disposed at the position P7 to thereby culture the cell again. The cell preservation apparatus 2 may be optionally configured to place the medium in advance into the dissociated cell collection section 48.

When the culture substrate 44 is irradiated with ultrasonic waves from the ultrasonic radiation section 1S to dissociate cells adhering to the culture substrate 44, and the cells having been dissociated from the culture substrate 44 include cell clumps, a treatment of separation into single cells may be performed. The treatment of separation into single cells can be performed by passing the cell dissociation solution containing cells (also serving as the cell preservation solution) thorough the mesh filter F2.

The cell preservation apparatus 2 may include the cell observation section 43 configured to capture an image of the cell cultured in the culture substrate 44, which enables examination of the state of cell dissociation or the state of cell collection.

The cell preservation apparatus 2 may include a controller configured to control the above-described cell preservation process. The controller is means for controlling operations of parts in the cell preservation apparatus 2. For example, the controller may be constituted by a computer including a processing section such as a CPU, a memory such as a RAM, and a storage section such as a hard disk drive. The controller may include an operation section. The operation section may include a display section and an input section, for example. The display section can display image data outputted from the controller or various information items related to operations of the cell preservation apparatus 2. For the display section, for example, a liquid crystal display is used. The input section receives various commands inputted by the user. For the input section, for example, a keyboard or a mouse is used. Both of the function of the display section and the function of the input section may be provided by a single unit such as a touch panel display.

Hereinafter, various measurement methods related to the present invention will be described.

### <Method of measuring molecular weight of polymer>

The molecular weights (such as peak molecular weight) of the polymer were measured by gel permeation chromatography (GPC) in the following manner. First, the polymer was dissolved in methanol at room temperature over 48 hours. Subsequently, the resultant solution was filtered through a solvent-resistant membrane filter having a pore size of 0.2 µm "Maishori disc H-25-2" (manufactured by Tosoh Corporation) to provide a sample solution. Note that the sample solution was prepared such that the concentration of methanol-soluble components became about 1.0 mass%. This sample solution was used and measured under the following conditions to determine the molecular weight distribution. Apparatus: Waters APC system (detector: RI) (manufactured by Waters Corporation)
Columns: ACQUITY APC XT900, XT200, XT125, and XT45 were connected (manufactured by Waters Corporation)
Eluent: methanol
Flow rate: 0.4 ml/min
Column temperature: 40.0°C
Sample injection amount: 0.01 ml

Various molecular weights of the sample were calculated using molecular weight calibration curves created using standard polyethylene glycol resin (for example, trade name "EasiVial PEG", manufactured by Agilent Technologies, Inc.).

### <Method of measuring viscosity of dissociation solution>

The viscosity of the dissociation solution was measured using an E-type viscometer RE-80L (manufactured by Toki Sangyo Co., Ltd.). The measurement method was as follows: 3 mL of the dissociation solution was placed into a measurement vessel and measured under conditions of a standard rotor at a shear rate of 100 rpm. Note that the measurement temperature was set at 37°C using a circulation thermostat connected.

### EXAMPLES

Hereinafter, the present invention will be described further in detail with reference to Examples and Comparative Examples, which do not limit the present invention at all.

### <Preparation example of dissociation solution 1>

To divalent-cation-free phosphate buffered saline (PBS(-), manufactured by Thermo Fisher Scientific Inc.), a polymer that was PEG2000 having a peak molecular weight Mp of 2030 as measured by GPC (manufactured by KISHIDA CHEMICAL Co., Ltd.) was added in 1.0 mass%, ethylenediaminetetraacetic acid was added in 0.1 mmol/L, and they were completely dissolved. Furthermore, hydrochloric acid or sodium hydroxide was used to adjust the pH to 7.4, and the resultant solution was used as a dissociation solution 1. The dissociation solution 1 was found to have a viscosity of 1.22 mPa·s.

### <Preparation examples of dissociation solutions 2 to 18>

The same procedures as in the dissociation solution 1 were performed except that the type of the polymer employed, mass% of the polymer, peak molecular weight Mp, the molarity of ethylenediaminetetraacetic acid, pH, and viscosity were changed as described in Table 1, to prepare dissociation solutions 2 to 18. Note that, for the dissociation solution 13, ethylenediaminetetraacetic acid was not added; for the dissociation solution 18, polyethylene glycol and ethylenediaminetetraacetic acid were not added.

### <Preparation example of dissociation solution 19>

To divalent-cation-free phosphate buffered saline (PBS(-), manufactured by Thermo Fisher Scientific Inc.), a polymer that was polyvinylpyrrolidone K15 having a peak molecular weight Mp of 11000 as measured by GPC (manufactured by Tokyo Chemical Industry Co., Ltd.) was added in 1.0 mass%, ethylenediaminetetraacetic acid was added in 0.1 mmol/L, and they were completely dissolved.

Furthermore, hydrochloric acid or sodium hydroxide was used to adjust the pH to 7.4, and the resultant solution was used as a dissociation solution 1. The dissociation solution 1 was found to have a viscosity of 1.19 mPa·s. Note that, in Table 1, polyvinylpyrrolidone is described as PVP.

### <Preparation example of dissociation solution 20>

To divalent-cation-free phosphate buffered saline (PBS(-), manufactured by Thermo Fisher Scientific Inc.), a polymer that was polyvinyl alcohol having a peak molecular weight Mp of 10500 as measured by GPC (manufactured by Sigma-Aldrich Corporation) was added in 1.0 mass%, ethylenediaminetetraacetic acid was added in 0.1 mmol/L, and they were completely dissolved.

Furthermore, hydrochloric acid or sodium hydroxide was used to adjust the pH to 7.4, and the resultant solution was used as a dissociation solution 1. The dissociation solution 1 was found to have a viscosity of 1.34 mPa·s. Note that, in Table 1, polyvinyl alcohol is described as PVA.

**[Table 1]**

| | Polymer | | | Ethylenediaminetetraacetic acid | pH | Viscosity mPa·s |
|---|---|---|---|---|---|---|
| | Type | mass% | Mp | Molarity mmol/L | | |
| Dissociation solution 1 | PEG | 1.0 | 2030 | 0.1 | 7.4 | 1.22 |
| Dissociation solution 2 | PEG | 0.03 | 2030 | 0.1 | 7.4 | 1.10 |
| Dissociation solution 3 | PEG | 0.1 | 2030 | 0.1 | 7.4 | 1.10 |
| Dissociation solution 4 | PEG | 5.0 | 2030 | 0.1 | 7.4 | 1.18 |
| Dissociation solution 5 | PEG | 20.0 | 2030 | 0.1 | 7.4 | 1.25 |
| Dissociation solution 6 | PEG | 1.0 | 860 | 0.1 | 7.4 | 1.19 |
| Dissociation solution 7 | PEG | 1.0 | 1200 | 0.1 | 7.4 | 1.20 |
| Dissociation solution 8 | PEG | 1.0 | 3700 | 0.1 | 7.4 | 1.23 |
| Dissociation solution 9 | PEG | 1.0 | 9100 | 0.1 | 7.4 | 1.36 |
| Dissociation solution 10 | PEG | 1.0 | 18000 | 0.1 | 7.4 | 1.45 |
| Dissociation solution 11 | PEG | 1.0 | 45000 | 0.1 | 7.4 | 1.81 |
| Dissociation solution 12 | PEG | 0.5 | 45000 | 0.1 | 7.4 | 1.49 |
| Dissociation solution 13 | PEG | 1.0 | 2030 | - | 7.4 | 1.22 |
| Dissociation solution 14 | PEG | 1.0 | 2030 | 0.01 | 7.4 | 1.22 |
| Dissociation solution 15 | PEG | 1.0 | 2030 | 5.0 | 7.4 | 1.22 |
| Dissociation solution 16 | PEG | 1.0 | 2030 | 0.1 | 6.5 | 1.22 |
| Dissociation solution 17 | PEG | 1.0 | 2030 | 0.1 | 8.0 | 1.22 |
| Dissociation solution 18 | - | - | - | - | 7.4 | 1.09 |
| Dissociation solution 19 | PVP | 1.0 | 11000 | 0.1 | 7.4 | 1.19 |
| Dissociation solution 20 | PVA | 1.0 | 10500 | 0.1 | 7.4 | 1.34 |

### [Example 1]

### (Culture of cells)

CHO (Chinese Hamster Ovary) cells were seeded in a Φ60 polystyrene dish (manufactured by Corning Incorporated) at a density of 10000 cells/cm², and cultured in an environment at 37°C and at a CO2 concentration of 5%. The medium was Ham's F12 (manufactured by Thermo Fisher Scientific Inc.) prepared so as to contain 10% of Fetal Bovine Serum (manufactured by Sigma-Aldrich Corporation) and 1% of penicillin-streptomycin (10000 U/ml, manufactured by Thermo Fisher Scientific Inc.). The cells were cultured for 48 hours; a phase-contrast microscope was used to observe the state of the cells and adhesion and growth of the cells were confirmed. The cell confluency in the dish was about 80%.

### (Dissociation of cells)

The medium in the dish was removed; the cells were washed with PBS(-) and subsequently immersed in the dissociation solution 1 for 3 minutes. Subsequently, an ultrasonic vibrator was brought into contact with the dish to apply, at an environmental temperature of 37°C, sweep vibration (frequency: 22-27 kHz, sweep period: 1 s, voltage: 200 V) for 3 minutes, to dissociate the cells. The dissociated cells were collected and subsequently subjected to measurement of the number of cells using a hemacytometer and a viability test using trypan blue staining to calculate viability. The dish having been subjected to the ultrasonic dissociation was treated with a cell scraper to dissociate all the cells not having been dissociated by ultrasonic waves, and a hemacytometer was used to measure the number of the cells. The number of the cells dissociated using ultrasonic waves and the number of the cells subsequently dissociated using the cell scraper were added up to provide the total number of dissociated cells; the ratio of the number of cells dissociated using ultrasonic waves to the total number of dissociated cells was defined as the dissociation ratio and calculated. As a result, the viability was 96.0% and the dissociation ratio was 98.0%. Note that Examples in which both of the viability and the dissociation ratio were 90% or more were evaluated as good.

### [Examples 2 to 18 and Comparative Examples 1, 3, and 4]

Dissociation and evaluations were performed by the same procedures as in Example 1 except that the type of the dissociation solution and the environmental temperature were changed. The results will be described in Table 2.

### [Example 19]

C2C12 cells from a mouse skeletal muscle myoblast cell line were seeded in a Φ60 polystyrene dish (manufactured by Corning Incorporated) at a density of 5000 cells/cm², and cultured in an environment at 37°C and at a CO2 concentration of 5%. The medium was DMEM/F12 (manufactured by Thermo Fisher Scientific Inc.) prepared so as to contain 10% of Fetal Bovine Serum (manufactured by Sigma-Aldrich Corporation) and 1% of penicillin-streptomycin (10000 U/ml, manufactured by Thermo Fisher Scientific Inc.). The cells were cultured for 48 hours; a phase-contrast microscope was used to observe the state of the cells and adhesion and growth of the cells were confirmed. The cell confluency in the dish was about 80%. Subsequent procedures of cell dissociation and evaluations were performed as in Example 1. The results will be described in Table 2.

### [Example 20]

A549 cells, which are human alveolar basement epithelium adenocarcinoma cells, were seeded in a Φ60 polystyrene dish (manufactured by Corning Incorporated) at a density of 10000 cells/cm², and cultured in an environment at 37°C and at a CO2 concentration of 5%. The medium employed was DMEM (manufactured by Thermo Fisher Scientific Inc.) prepared so as to contain 10% of Fetal Bovine Serum (manufactured by Sigma-Aldrich Corporation) and 1% of penicillin-streptomycin (10000 U/ml, manufactured by Thermo Fisher Scientific Inc.). The cells were cultured for 48 hours; a phase-contrast microscope was used to observe the state of the cells and adhesion and growth of the cells were confirmed. The cell confluency in the dish was about 80%. Subsequent procedures of cell dissociation and evaluations were performed as in Example 1. The results will be described in Table 2.

### [Example 21]

HUVEC (Human Umbilical Vein Endothelial Cells) cells were seeded in a Φ60 polystyrene dish (manufactured by Corning Incorporated) at a density of 10000 cells/cm², and cultured in an environment at 37°C and at a CO2 concentration of 5%. The medium employed was Endothelial Cell Growth Medium 2 Kit (manufactured by PromoCell GmbH). The cells were cultured for 48 hours; a phase-contrast microscope was used to observe the state of the cells and adhesion and growth of the cells were confirmed. The cell confluency in the dish was about 80%. Subsequent procedures of cell dissociation and evaluations were performed as in Example 1. The results will be described in Table 2.

### [Example 22]

hMSC (Human Mesenchymal Stem Cells) cells were seeded in a Φ60 polystyrene dish (manufactured by Corning Incorporated) at a density of 4000 cells/cm², and cultured in an environment at 37°C and at a CO2 concentration of 5%. The medium employed was MSCGM Bullet Kit (manufactured by Lonza). After culture was performed for 96 hours, the medium was exchanged and subsequently culture was performed to reach 168 hours. A phase-contrast microscope was used to observe the state of the cells and adhesion and growth of the cells were confirmed. Subsequent procedures of cell dissociation and evaluations were performed as in Example 1. The cell confluency in the dish was about 80%. The results will be described in Table 2.

### [Comparative Example 2]

The same procedures as in Example 21 were performed except that the dissociation solution used was changed to the dissociation solution 18. The results will be described in Table 2.

**[Table 2]**

| | Type of dissociation solution | Type of cells | Environmental temperature | Viability % | Dissociation ratio % |
|---|---|---|---|---|---|
| Example 1 | Dissociation solution 1 | CHO | 37.0 | 96.0 | 98.0 |
| Example 2 | Dissociation solution 2 | CHO | 37.0 | 92.7 | 97.8 |
| Example 3 | Dissociation solution 3 | CHO | 37.0 | 94.6 | 97.9 |
| Example 4 | Dissociation solution 4 | CHO | 37.0 | 96.2 | 97.5 |
| Example 5 | Dissociation solution 5 | CHO | 37.0 | 95.8 | 97.2 |
| Example 6 | Dissociation solution 6 | CHO | 37.0 | 93.8 | 98.3 |
| Example 7 | Dissociation solution 7 | CHO | 37.0 | 95.3 | 98.2 |
| Example 8 | Dissociation solution 8 | CHO | 37.0 | 95.7 | 98.0 |
| Example 9 | Dissociation solution 9 | CHO | 37.0 | 95.6 | 97.9 |
| Example 10 | Dissociation solution 10 | CHO | 37.0 | 95.4 | 97.8 |
| Example 11 | Dissociation solution 11 | CHO | 37.0 | 93.9 | 93.4 |
| Example 12 | Dissociation solution 12 | CHO | 37.0 | 94.0 | 97.6 |
| Example 13 | Dissociation solution 13 | CHO | 37.0 | 95.8 | 93.2 |
| Example 14 | Dissociation solution 14 | CHO | 37.0 | 96.0 | 95.0 |
| Example 15 | Dissociation solution 15 | CHO | 37.0 | 95.5 | 98.3 |
| Example 16 | Dissociation solution 16 | CHO | 37.0 | 95.9 | 95.2 |
| Example 17 | Dissociation solution 17 | CHO | 37.0 | 95.5 | 98.8 |
| Example 18 | Dissociation solution 1 | CHO | 25.0 | 94.0 | 94.2 |
| Example 19 | Dissociation solution 1 | C2C12 | 37.0 | 95.8 | 97.9 |
| Example 20 | Dissociation solution 1 | A549 | 37.0 | 96.0 | 95.8 |
| Example 21 | Dissociation solution 1 | HUVEC | 37.0 | 95.3 | 94.8 |
| Example 22 | Dissociation solution 1 | hMSC | 37.0 | 94.8 | 98.0 |
| Comparative Example 1 | Dissociation solution 18 | CHO | 37.0 | 89.8 | 93.3 |
| Comparative Example 2 | Dissociation solution 18 | HUVEC | 37.0 | 87.0 | 88.0 |
| Comparative Example 3 | Dissociation solution 19 | CHO | 37.0 | 89.1 | 97.5 |
| Comparative Example 4 | Dissociation solution 20 | CHO | 37.0 | 89.9 | 97.1 |

The present invention can provide a cell dissociation solution and a cell dissociation method that provide, in cell dissociation using ultrasonic waves, both of a high cell viability and a high dissociation ratio.

### [Example 23]

### (Culture of cells on substrate)

hMSC (Human Mesenchymal Stem Cells) cells were seeded in a Φ60 polystyrene dish (manufactured by Corning Incorporated) at a density of 4000 cells/cm2, and cultured in an environment at 37°C and at a CO2 concentration of 5%. The medium employed was MSCGM Bullet Kit (manufactured by Lonza). After culture was performed for 96 hours, the medium was exchanged and subsequently culture was performed to reach 168 hours. A phase-contrast microscope was used to observe the state of the cells and adhesion and growth of the cells were confirmed. The cell confluency in the dish was about 80%.

### (Dissociation of cells from substrate)

The medium in the dish was removed; the cells were washed with PBS(-) and subsequently immersed in the dissociation solution 1 for 10 minutes. Subsequently, an ultrasonic vibrator was brought into contact with the dish to apply, at an environmental temperature of 37°C, sweep vibration (frequency: 22-27 kHz, sweep period: 1 s, voltage: 100 V) for 3 minutes, to dissociate the cells. The dissociated cells were collected and adjusted to a cell density of 1.0 × 10⁶ cells/mL.

### (Cold preservation of cells)

The dissociation solution 1 containing cells was placed into a polypropylene tube and sealed. This dissociated cell preservation vessel was preserved in an environment at 4°C (cold preservation). The cell viability relative to the preservation period during cell preservation was measured. The cell viability was measured by a viability test using trypan blue staining. After preservation for 1 day, the cell viability was 80.5%. After preservation for 3 days, the cell viability was 62.1%. Such results have demonstrated that such a cell preservation method according to the present invention enables cold preservation of cells. The cell preservation method according to the present invention has been demonstrated to be simple and effective.

### [Comparative Example 5]

Cold preservation of dissociated cells was performed as in Example 23 except that the cells were dissociated from the substrate not using ultrasonic vibration, but using trypsin as the dissociation solution, and the cell viability relative to the preservation period of the cells was measured. Note that, after dissociation from the substrate using trypsin, trypsin was neutralized using a serum medium; centrifugation was performed and the cells were dispersed again using PBS(-) to provide a preservation solution. As a result, the cell viability after preservation for 1 day was 77.1%. The cell viability after preservation for 3 days was 41.3%. Compared with Example 23, the cell viability after preservation for 3 days considerably decreased.

### [Example 24]

In Example 23, the cells having been subjected to cold preservation for 3 days were seeded in a polystyrene multiple plate (manufactured by Corning Incorporated) at a density of 4000 cells/cm2 and cultured in an environment at 37°C and at a CO2 concentration of 5%. Microscopic observation of growth of the cultured cells has demonstrated that the growth of the cells is similar to the pre-preservation growth. This result has demonstrated that cells provided by such a cell preservation method according to the present invention still have the cell growth capacity even after preservation using the dissociation solution 1, and the cells can be continuously cultured.

The disclosure of the present invention in this Description includes the following.

### [Embodiment 1]

A cell dissociation solution used in dissociation of, from a substrate, a cell disposed on the substrate, by application of ultrasonic vibration to the cell, the cell dissociation solution containing a polymer including a polyalkylene glycol structure.

### [Embodiment 2]

The cell dissociation solution according to Embodiment 1, wherein a content of the polymer relative to a total mass of the cell dissociation solution is 0.03 mass% or more and 20.0 mass% or less.

### [Embodiment 3]

The cell dissociation solution according to Embodiment 1 or 2, wherein a content of the polymer relative to a total mass of the cell dissociation solution is 0.05 mass% or more and 5.0 mass% or less.

### [Embodiment 4]

The cell dissociation solution according to any one of Embodiments 1 to 3, wherein the polymer has a peak molecular weight Mp of 800 or more and 50000 or less as measured by gel permeation chromatography.

### [Embodiment 5]

The cell dissociation solution according to any one of Embodiments 1 to 4, wherein the polymer has a peak molecular weight Mp of 1200 or more and 20000 or less as measured by gel permeation chromatography.

### [Embodiment 6]

The cell dissociation solution according to any one of Embodiments 1 to 5, wherein the polymer is polyethylene glycol.

### [Embodiment 7]

The cell dissociation solution according to any one of Embodiments 1 to 6, wherein the cell dissociation solution is substantially free from protease.

### [Embodiment 8]

The cell dissociation solution according to Embodiment 7, wherein the protease is trypsin.

### [Embodiment 9]

The cell dissociation solution according to any one of Embodiments 1 to 8, wherein the cell dissociation solution is substantially free from divalent cations.

### [Embodiment 10]

The cell dissociation solution according to any one of Embodiments 1 to 9, wherein the cell dissociation solution is substantially free from Ca2+ and Mg2+.

### [Embodiment 11]

The cell dissociation solution according to any one of Embodiments 1 to 10, wherein the cell dissociation solution has a pH of 7.0 or more and 8.0 or less.

### [Embodiment 12]

The cell dissociation solution according to any one of Embodiments 1 to 11, further containing a chelating agent, wherein the chelating agent has a concentration of 0.01 mM or more and 5.0 mM or less.

### [Embodiment 13]

The cell dissociation solution according to Embodiment 12, wherein the chelating agent is ethylenediaminetetraacetic acid.

### [Embodiment 14]

The cell dissociation solution according to any one of Embodiments 1 to 13, wherein the cell dissociation solution has a viscosity at 37°C of 1.80 mPa·s or less.

### [Embodiment 15]

A cell dissociation method for dissociating a cell disposed on a substrate, the cell dissociation method including:
a first step of preparing the substrate on which the cell and a cell dissociation solution in contact with the cell and the substrate are disposed; and
a second step of applying ultrasonic vibration to the cell to dissociate the cell from the substrate,
wherein the cell dissociation solution contains a polymer and the polymer is a polymer including a polyalkylene glycol structure.

### [Embodiment 16]

The cell dissociation method according to Embodiment 15, wherein the second step is performed at an environmental temperature of 30.0°C or more and 37.5°C or less.

### [Embodiment 17]

A cell preservation method for preserving a cell disposed on a substrate, the cell preservation method including:
(1) a step 1 of bringing the cell into contact with the cell dissociation solution according to Embodiments 1 to 14;
(2) a step 2 of applying ultrasonic vibration to the cell to dissociate the cell from the substrate; and
(3) a step 3 of preserving, in the cell dissociation solution, the cell having been dissociated in the step 2.

### [Embodiment 18]

The cell preservation method according to Embodiment 17, wherein the step 3 is non-cryopreservation.

### [Embodiment 19]

The cell preservation method according to Embodiments 17 to 18, wherein the cell is a stem cell.

### [Embodiment 20]

A cell culture method including seeding, to a cell growth medium, the cell having been preserved by the cell preservation method according to any one of Embodiments 17 to 19.

### [Embodiment 21]

A cell preservation apparatus configured to preserve a cell disposed on a substrate, the cell preservation apparatus including:
a medium exchange section configured to add, to the substrate to which the cell adheres, the cell dissociation solution according to Embodiments 1 to 14 to bring the cell and the cell dissociation solution into contact with each other;
an ultrasonic radiation section configured to apply ultrasonic vibration to the cell; and
a cell preservation section configured to preserve the cell having been dissociated from the substrate.

The present invention is not limited to the above-described embodiments, and various changes and modifications can be made without departing from the spirit and scope of the present invention. Thus, in order to apprise the public of the scope of the present invention, the following Claims are attached.

This application claims priority to Japanese Patent Application No. 2021-177910 filed October 29, 2021, which is hereby incorporated by reference herein in its entirety.

## Claims

1. A cell dissociation solution used in dissociation of, from a substrate, a cell disposed on the substrate, by application of ultrasonic vibration to the cell, the cell dissociation solution comprising a polymer including a polyalkylene glycol structure.

2. The cell dissociation solution according to claim 1, wherein a content of the polymer relative to a total mass of the cell dissociation solution is 0.03 mass% or more and 20.0 mass% or less.

3. The cell dissociation solution according to claim 1 or 2, wherein a content of the polymer relative to a total mass of the cell dissociation solution is 0.05 mass% or more and 5.0 mass% or less.

4. The cell dissociation solution according to any one of claims 1 to 3, wherein the polymer has a peak molecular weight Mp of 800 or more and 50000 or less as measured by gel permeation chromatography.

5. The cell dissociation solution according to any one of claims 1 to 4, wherein the polymer has a peak molecular weight Mp of 1200 or more and 20000 or less as measured by gel permeation chromatography.

6. The cell dissociation solution according to any one of claims 1 to 5, wherein the polymer is polyethylene glycol.

7. The cell dissociation solution according to any one of claims 1 to 6, wherein the cell dissociation solution is substantially free from protease.

8. The cell dissociation solution according to claim 7, wherein the protease is trypsin.

9. The cell dissociation solution according to any one of claims 1 to 8, wherein the cell dissociation solution is substantially free from divalent cations.

10. The cell dissociation solution according to any one of claims 1 to 9, wherein the cell dissociation solution is substantially free from Ca²⁺ and Mg²⁺.

11. The cell dissociation solution according to any one of claims 1 to 10, wherein the cell dissociation solution has a pH of 7.0 or more and 8.0 or less.

12. The cell dissociation solution according to any one of claims 1 to 11, further comprising a chelating agent, wherein the chelating agent has a concentration of 0.01 mM or more and 5.0 mM or less.

13. The cell dissociation solution according to claim 12, wherein the chelating agent is ethylenediaminetetraacetic acid.

14. The cell dissociation solution according to any one of claims 1 to 13, wherein the cell dissociation solution has a viscosity at 37°C of 1.80 mPa·s or less.

15. A cell dissociation method for dissociating a cell disposed on a substrate, the cell dissociation method comprising:
a first step of preparing the substrate on which the cell and a cell dissociation solution in contact with the cell and the substrate are disposed; and
a second step of applying ultrasonic vibration to the cell to dissociate the cell from the substrate,
wherein the cell dissociation solution contains a polymer and the polymer is a polymer including a polyalkylene glycol structure.

16. The cell dissociation method according to claim 15, wherein the second step is performed at an environmental temperature of 30.0°C or more and 37.5°C or less.

17. A cell preservation method for preserving a cell disposed on a substrate, the cell preservation method comprising:
(1) a step 1 of bringing the cell into contact with the cell dissociation solution according to claims 1 to 14;
(2) a step 2 of applying ultrasonic vibration to the cell to dissociate the cell from the substrate; and
(3) a step 3 of preserving, in the cell dissociation solution, the cell having been dissociated in the step 2.

18. The cell preservation method according to claim 17, wherein the step 3 is non-cryopreservation.

19. The cell preservation method according to claims 17 to 18, wherein the cell is a stem cell.

20. A cell culture method comprising seeding, to a cell growth medium, the cell having been preserved by the cell preservation method according to any one of claims 17 to 19.

21. A cell preservation apparatus configured to preserve a cell disposed on a substrate, the cell preservation apparatus comprising:
a medium exchange section configured to add, to the substrate to which the cell adheres, the cell dissociation solution according to claims 1 to 14 to bring the cell and the cell dissociation solution into contact with each other;
an ultrasonic radiation section configured to apply ultrasonic vibration to the cell; and
a cell preservation section configured to preserve the cell having been dissociated from the substrate.
